# FASCICULE DE BREVET EUROPEEN

(11) **EP 1 683 501 B1**
(45) Date de publication et mention de la délivrance du brevet: **29.10.2008**
(21) Numéro de dépôt: 06356005.6
(22) Date de dépôt: 10.01.2006
(51) Int. Cl.: A61F 2/40, A61B 17/82

(54) **Dispositif d'osteosynthese pour articulation de l'epaule**
Osteosynthesevorrichtung für ein Schultergelenk
Device for the osteosynthesis of a shoulder joint

(30) Priorité: 20.01.2005 FR 0500565
(43) Date de publication de la demande: 26.07.2006
(73) Titulaire: Groupe Lepine, 69394 Lyon Cedex 03 (FR)
(72) Inventeur: Chirpaz-Cerbat, Jean Marie, 74000 Annency (FR); Martinez, Thierry, 73310 Serrieres en Chautagne (FR); Saragaglia, Dominique, 38640 Claix (FR)
(74) Mandataire: Jeannet, Olivier

(56) Documents cités:
- US-A- 4 045 825
- US-A- 5 611 801
- US-A- 5 697 934
- US-A- 5 993 452
- US-B1- 6 171 341

## Description

La présente invention concerne un dispositif d'ostéosynthèse pour articulation de l'épaule. Elle concerne également une prothèse d'épaule utilisable avec ce dispositif d'ostéosynthèse.

Une fracture de l'articulation de l'épaule peut conduire, particulièrement en cas d'ostéoporose, à une rupture de la partie supérieure de l'humérus en trois ou quatre fragments, à savoir la tête humérale, les tubérosités, c'est-à-dire le trochin et le trochiter, et la diaphyse. Les deux tubérosités peuvent elles-mêmes être séparées l'une de l'autre.

Une technique classique de traitement d'une fracture de l'épaule consiste à mettre en place une prothèse d'épaule comprenant une tête humérale prothétique, avec positionnement d'un greffon osseux sous cette tête prothétique, du côté externe, puis à opérer une synthèse des tubérosités et de l'humérus, en maintenant les tubérosités contre ce greffon.

La croissance des cellules osseuses permet de ressouder les tubérosités au reste de l'humérus. Il arrive cependant que la soudure osseuse ne s'opère pas de manière optimale, ou que le patient entreprenne une rééducation trop précoce, alors que la consolidation osseuse n'est pas encore suffisante. Compte tenu des efforts exercés sur les tubérosités par les muscles reliés à elles, le risque existe alors que se produise une désolidarisation des tubérosités.

Les documents US 5,611,801, US 5,993,452 et US 5,697,934 décrivent différents types de matériels pouvant notamment être utilisés pour traiter des fractures intervenant au niveau des tubérosités d'une épaule.

Ces matériels n'apparaissent cependant pas donner parfaitement satisfaction en ce qui concerne le traitement de telles fractures.

La présente invention vise à remédier à cet inconvénient essentiel.

Son objectif principal est de fournir un dispositif d'ostéosynthèse implantable permettant de supprimer le risque d'une désolidarisation des tubérosités et donc d'assurer que la consolidation de l'humérus se produise dans les meilleures conditions.

A cet effet, le dispositif d'ostéosynthèse selon l'invention comprend une pièce d'enveloppement présentant une longueur et une largeur telles qu'elle peut envelopper l'ensemble des tubérosités, et des moyens de maintien de cette pièce d'enveloppement appliquée sur les tubérosités.

Le dispositif selon l'invention permet ainsi de plaquer les tubérosités contre l'humérus et de les immobiliser par rapport à l'humérus. Il assure une prévention efficace de tout risque de désolidarisation des tubérosités et permet par conséquent une consolidation osseuse dans les meilleures conditions.

Ladite pièce d'enveloppement a de préférence une forme courbe d'une extrémité longitudinale à l'autre, qui facilite sa mise en place sur les tubérosités et lui permet d'assurer un parfait enveloppement, et donc un parfait maintien, des tubérosités.

Dans le même but, cette pièce d'enveloppement peut également avoir une forme concave au niveau de sa face destinée à être appliquée contre les tubérosités.

De préférence, ladite pièce d'enveloppement est dimensionnée pour s'étendre jusqu'au niveau des faces métaphysaires antérieures et postérieures de l'humérus.

Une parfaite immobilisation de cette pièce par rapport à l'os est ainsi obtenue.

La pièce d'enveloppement peut présenter une longueur telle que ses extrémités longitudinales viennent à proximité l'une de l'autre au niveau de la face interne de l'humérus.

Cette pièce d'enveloppement présente avantageusement une largeur allant en se réduisant progressivement de sa zone médiane en direction de ses extrémités longitudinales.

Cette forme effilée de la pièce d'enveloppement est bien adaptée à l'anatomie de la partie métaphysaire de l'humérus.

Selon une possibilité, la pièce d'enveloppement est en un matériau ajouré souple. Un tel matériau confère à la pièce d'enveloppement une déformabilité lui permettant de parfaitement épouser la forme des tubérosités.

Ce matériau peut notamment être sous forme d'un filet, et ce filet peut comprendre des fils longitudinaux s'étendant d'une extrémité longitudinale à l'autre de la pièce d'enveloppement, et convergeant les uns vers les autres au niveau de chacune de ces extrémités longitudinales.

Le filet peut notamment être formé à partir de fils en matériaux hyperélastiques, notamment en alliage nickel-titane tel que celui connu sous la dénomination "Nitinol", ou en acier inoxydable, ou en matériau résorbable.

Selon une autre forme de réalisation de l'invention, la pièce d'enveloppement est formée par une coquille en un matériau peu déformable, par exemple en acier inoxydable.

Lesdits moyens de maintien peuvent être formés par une structure légèrement élastique de la pièce d'enveloppement, celle-ci ayant alors des parties venant en application avec frottements contre l'os, et/ou peuvent être formés par au moins deux brins latéraux, reliés chacun à l'une des extrémités longitudinales de ladite pièce d'enveloppement, ces brins pouvant être reliés l'un à l'autre afin d'assurer le maintien de la pièce d'enveloppement en position d'application contre les tubérosités.

La prothèse d'épaule utilisable avec le dispositif d'ostéosynthèse selon invention peut alors comprendre des moyens pour l'accrochage d'au moins un desdits brins latéraux, tels que des rainures ou des trous.

Pour sa bonne compréhension, l'invention est à nouveau décrite ci-dessous, en référence au dessin schématique annexé représentant, à titre d'exemples non limitatifs, deux formes de réalisation possibles du dispositif d'ostéosynthèse pour articulation de l'épaule, qu'elle concerne.
La figure 1 en est une vue en perspective, selon une première forme de réalisation ;
la figure 2 en est une vue de dessus ;
la figure 3 en est une vue de côté ;
la figure 4 en est une vue de côté, après mise en place sur un humérus ;
la figure 5 en est une vue de face, selon une deuxième forme de réalisation, et
la figure 6 en est une vue de côté, après mise en place sur un humérus.

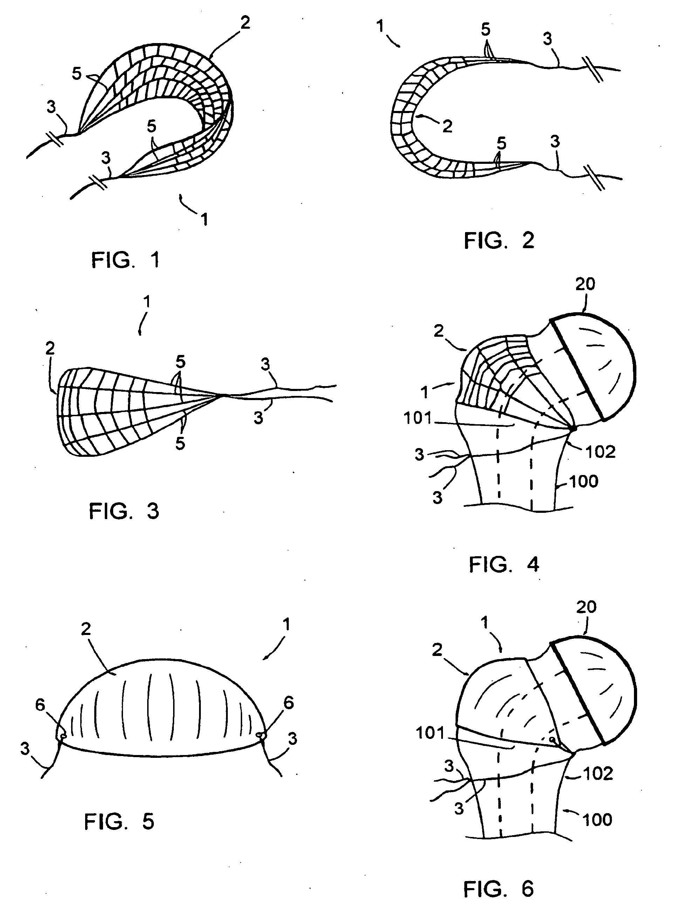

Les figures 1 à 3 représentent un dispositif d'ostéosynthèse 1 pour articulation de l'épaule, comprenant une pièce d'enveloppement 2 et deux brins latéraux de maintien 3.

La pièce 2 est formée par un matériau ajouré souple, notamment un filet réalisé par tissage ou tricotage de fils en alliage nickel-titane connu sous la dénomination "Nitinol". Ce filet comprend des fils longitudinaux 5 s'étendant d'une extrémité longitudinale à l'autre de la pièce d'enveloppement 2, et convergeant les uns vers les autres au niveau de chacune des extrémités longitudinales de cette pièce 2, de sorte que cette dernière présente une largeur allant en se réduisant progressivement de sa zone médiane en direction de ses extrémités longitudinales.

La pièce d'enveloppement 2 a une forme courbe d'une extrémité longitudinale à l'autre (cf. figure 2) et une forme concave au niveau de sa face tournée vers l'intérieur de sa courbure (cf. figure 3).

La pièce d'enveloppement 2 a en outre une longueur telle qu'elle peut s'étendre, lorsqu'elle est mise en place sur un humérus 100 ainsi que le montre la figure 4, jusqu'au niveau des faces métaphysaires 101 antérieures et postérieures de l'humérus 100, le long de la tête humérale prothétique 20 d'une prothèse d'épaule mise en place sur l'humérus 100. Les extrémités longitudinales de la pièce 2 viennent à proximité l'une de l'autre au niveau de la face interne 102 de l'humérus, en dessous de la tête 20. Ces extrémités peuvent venir au contact de la tige de la prothèse, qui peut présenter des formes d'accroche spécifiques telles que des rainures ou des trous.

Les brins latéraux 3 sont en matériau résistant et sont tels qu'ils peuvent être reliés l'un à l'autre, par nouage ou torsade. Ces brins 3 peuvent notamment être constitués par la prolongation d'un ou plusieurs des fils 5.

En pratique, comme le montre la figure 4, le dispositif 1 est mis en place sur un humérus 100 de telle sorte que la pièce d'enveloppement 2 recouvre l'ensemble des tubérosités. Grâce à sa forme précitée, cette pièce 2 est bien adaptée à la forme anatomique de la partie métaphysaire proximale de l'humérus 100. Les extrémités longitudinales de la pièce 2 viennent à proximité l'une de l'autre, au niveau de la face interne 102, et les brins 3 sont croisés à cet endroit puis ramenés au niveau de la face externe de l'humérus 100, au niveau de laquelle ils sont reliés l'un à l'autre.

La pièce 2 est ainsi parfaitement maintenue par rapport à l'humérus 100, en étant appliquée étroitement sur les tubérosités.

Selon une autre forme de réalisation, comme le montrent les figures 5 et 6, la pièce d'enveloppement 2 est formée par une coquille en un matériau plein et peu déformable, par exemple en acier inoxydable. Dans ce cas, la pièce 2 comprend deux trous 6 au niveau de ses extrémités longitudinales, au travers desquels sont engagés les brins 3.

Comme cela apparaît de ce qui précède, l'invention fournit un dispositif d'ostéosynthèse pour articulation de l'épaule, présentant les avantages déterminants de permettre de plaquer les tubérosités contre l'humérus et de les immobiliser parfaitement par rapport à l'humérus. Ce dispositif permet par conséquent une consolidation osseuse dans les meilleures conditions et une prévention efficace de tout risque de migration des tubérosités tant que cette consolidation osseuse n'est pas accomplie.

Il va de soi que l'invention n'est pas limitée à la forme de réalisation de l'invention décrite ci-dessus à titre d'exemple mais qu'elle s'étend à toutes les variantes de réalisation couverte par les revendications ci-annexées.

## Revendications

1. Dispositif d'ostéosynthèse (1) pour articulation de l'épaule, comprenant une pièce (2) de liaison des tubérosités au reste de l'humérus et des moyens (3) de maintien de cette pièce de liaison, **caractérisé en ce que** ladite pièce (2) de liaison est une pièce d'enveloppement (2) présentant une longueur et une largeur telles qu'elle peut envelopper l'ensemble des tubérosités, en étant appliquée étroitement sur ces tubérosités, de manière à parfaitement épouser la forme de celles-ci, et **en ce que** lesdits moyens (3) de maintien permettent de maintenir la pièce d'enveloppement appliquée sur les tubérosités.

2. Dispositif d'ostéosynthèse (1) selon la revendication 1, **caractérisé en ce que** la pièce d'enveloppement (2) a une forme courbe d'une extrémité longitudinale à l'autre.

3. Dispositif d'ostéosynthèse (1) selon la revendication 1 ou la revendication 2, **caractérisé en ce que** la pièce d'enveloppement (2) a une forme concave au niveau de sa face destinée à être appliquée contre les tubérosités.

4. Dispositif d'ostéosynthèse (1) selon l'une des revendications 1 à 3, **caractérisé en ce que** la pièce d'enveloppement (2) est dimensionnée pour s'étendre jusqu'au niveau des faces métaphysaires (101) antérieures et postérieures de l'humérus (100).

5. Dispositif d'ostéosynthèse (1) selon l'une des revendications 1 à 4, **caractérisé en ce que** la pièce d'enveloppement (2) présente une longueur telle que ses extrémités longitudinales viennent à proximité l'une de l'autre au niveau de la face interne (102) de l'humérus (100).

6. Dispositif d'ostéosynthèse (1) selon l'une des revendications 1 à 5, **caractérisé en ce que** la pièce d'enveloppement (2) présente une largeur allant en se réduisant progressivement de la zone médiane de cette pièce d'enveloppement (2) en direction des extrémités longitudinales de celle-ci.

7. Dispositif d'ostéosynthèse (1) selon l'une des revendications 1 à 6, **caractérisé en ce que** la pièce d'enveloppement (2) est en un matériau ajouré souple.

8. Dispositif d'ostéosynthèse (1) selon la revendication 7, **caractérisé en ce que** le matériau ajouré souple est sous forme d'un filet, et **en ce que** ce filet comprend des fils longitudinaux (5) s'étendant d'une extrémité longitudinale à l'autre de la pièce d'enveloppement (2), et convergeant les uns vers les autres au niveau de chacune de ces extrémités longitudinales.

9. Dispositif d'ostéosynthèse (1) selon la revendication 8, **caractérisé en ce que** le filet est formé à partir de fils en matériaux hyperélastiques, notamment en alliage nickel-titane tel que celui connu sous la dénomination "Nitinol", ou en acier inoxydable, ou en matériau résorbable.

10. Dispositif d'ostéosynthèse (1) selon l'une des revendications 1 à 6, **caractérisé en ce que** la pièce d'enveloppement (2) est formée par une coquille en un matériau peu déformable, par exemple en acier inoxydable.

11. Dispositif d'ostéosynthèse (1) selon l'une des revendications 1 à 10, **caractérisé en ce que** lesdits moyens de maintien sont formés par une structure légèrement élastique de la pièce d'enveloppement, celle-ci ayant alors des parties venant en application avec frottements contre l'os, et/ou peuvent être formés par au moins deux brins latéraux (3); reliés chacun à l'une des extrémités longitudinales de ladite pièce d'enveloppement, ces brins pouvant être reliés l'un à l'autre afin d'assurer le maintien de la pièce d'enveloppement en position d'application contre les tubérosités.

12. Prothèse d'épaule en combinaison avec le dispositif d'ostéosynthèse selon la revendication 11, **caractérisée en ce qu'**elle comprend des moyens pour l'accrochage d'au moins un desdits brins latéraux (3), tels que des rainures ou des trous.

## Claims

1. An osteosynthesis device (1) for articulation of the shoulder, comprising a piece (2) for connecting tuberosities to the rest of the humerus and means (3) for maintaining this connecting piece, **characterized in that** said connecting piece (2) is a covering piece (2) having a length and width such that it can envelop all of the tuberosities, being applied closely on these tuberosities, so as to perfectly fit the shape thereof, and **in that** said maintenance means (3) make it possible to maintain the covering piece applied on the tuberosities.

2. The osteosynthesis device (1) according to claim 1, **characterized in that** the covering piece (2) has a curved shape from one longitudinal end to the other.

3. The osteosynthesis device (1) according to claim 1 or claim 2, **characterized in that** the covering piece (2) has a concave shape at its surface designed to be applied against the tuberosities.

4. The osteosynthesis device (1) according to one of claims 1 to 3, **characterized in that** the covering piece (2) is sized to extend to the anterior and posterior metaphyseal surfaces (101) of the humerus (100).

5. The osteosynthesis device (1) according to one of claims 1 to 4, **characterized in that** the covering piece (2) has a length such that its longitudinal ends come close to each other at the internal surface (102) of the humerus (100).

6. The osteosynthesis device (1) according to one of claims 1 to 5, **characterized in that** the covering piece (2) has a width which progressively decreases from the median area of this covering piece (2) toward the longitudinal ends thereof.

7. The osteosynthesis device (1) according to one of claims 1 to 6, **characterized in that** the covering piece (2) is made in a flexible openwork material.

8. The osteosynthesis device (1) according to claim 7, **characterized in that** the flexible openwork material is in the form of a net, and **in that** this net comprises longitudinal threads (5) extending from one longitudinal end of the covering piece (2) to the other, and converging toward each other at each of these longitudinal ends.

9. The osteosynthesis device (1) according to claim 8, **characterized in that** the net is formed from threads in hyperelastic materials, in particular in a nickel-titanium alloy such as that known by the name "Nitinol", or in stainless steel, or in a resorptive material.

10. The osteosynthesis device (1) according to one of claims 1 to 6, **characterized in that** the covering piece (2) is formed by a shell in a low-distortion material, for example in stainless steel.

11. The osteosynthesis device (1) according to one of claims 1 to 10, **characterized in that** said maintenance means are formed by a slightly elastic structure of the covering piece, this then having parts frictionally applied against the bone, and/or can be formed by at least two lateral strands (3), each connected to one of the longitudinal ends of said covering piece, these strands being able to be connected to each other in order to ensure that the covering piece is maintained applied against the tuberosities.

12. A shoulder prosthesis in combination with the osteosynthesis device according to claim 11, **characterized in that** it comprises means for attaching at least one of said lateral strands (3), such as grooves or holes.

## Patentansprüche

1. Osteosynthesevorrichtung (1) für Schultergelenk, ein Teil (2) zur Verbindung der Tuberositates mit dem Rest des Humerus umfassend und Mittel (3) zum Halten dieses Verbindungsteils, **dadurch gekennzeichnet, dass** das Verbindungsteil (2) ein Umhüllungsteil (2) ist, das eine Länge und eine Breite aufweist, die derart sind, dass die Tuberositates insgesamt von ihm umhüllbar sind, wenn es eng auf diesen Tuberositates anliegt, in der Art, dass es ihre Form genauestens annimmt, und **dadurch**, dass die Haltemittel (3) erlauben, das Umhüllungsteil auf den Tuberositates anliegend zu halten.

2. Osteosynthesevorrichtung (1) nach Anspruch 1, **dadurch gekennzeichnet, dass** das Umhüllungsteil (2) eine von einem Längsende zum anderen gekrümmte Form hat.

3. Osteosynthesevorrichtung (1) nach Anspruch 1 oder Anspruch 2, **dadurch gekennzeichnet, dass** das Umhüllungsteil (2) auf Ebene seiner Seite, die an den Tuberositates anliegt, eine konkave Form hat.

4. Osteosynthesevorrichtung (1) nach einem der Ansprüche 1 bis 3, **dadurch gekennzeichnet, dass** das Umhüllungsteil (2) bemessen ist, um sich bis zur Ebene der metaphysären Vorder- und Hinterseiten (101) des Humerus (100) zu erstrecken.

5. Osteosynthesevorrichtung (1) nach einem der Ansprüche 1 bis 4, **dadurch gekennzeichnet, dass** das Umhüllungsteil (2) eine Länge aufweist, die derart ist, dass sich seine Längsenden an der Innenseite (102) des Humerus einander annähern.

6. Osteosynthesevorrichtung (1) nach einem der Ansprüche 1 bis 5, **dadurch gekennzeichnet, dass** das Umhüllungsteil (2) eine Breite aufweist, die, indem sie sich schrittweise vom mittleren Bereich dieses Umhüllungsteils (2) reduziert, in Richtung seiner Längsenden geht.

7. Osteosynthesevorrichtung (1) nach einem der Ansprüche 1 bis 6, **dadurch gekennzeichnet, dass** das Umhüllungsteil (2) aus einem elastischen durchbrochenen Material ist.

8. Osteosynthesevorrichtung (1) nach Anspruch 7, **dadurch gekennzeichnet, dass** das elastische durchbrochene Material in Form eines Netzes ist, und **dadurch**, dass dieses Netz Längsfäden (5) umfasst, die sich von einem Längsende des Umhüllungsteils (2) zum anderen erstrecken und an diesen Längsenden zusammenlaufen.

9. Osteosynthesevorrichtung (1) nach Anspruch 8, **dadurch gekennzeichnet, dass** das Netz aus Fäden aus hyperelastischen Materialen gebildet ist, vor allem aus einer Nickel-Titan-Legierung wie derjenigen, die unter der Bezeichnung "Nitinol" bekannt ist, oder aus Edelstahl oder aus einem resorbierbaren Material.

10. Osteosynthesevorrichtung (1) nach einem der Ansprüche 1 bis 6, **dadurch gekennzeichnet, dass** das Umhüllungsteil (2) von einer Schale aus einem wenig deformierbaren Material, zum Beispiel aus Edelstahl, gebildet ist.

11. Osteosynthesevorrichtung (1) nach einem der Ansprüche 1 bis 10, **dadurch gekennzeichnet, dass** die Haltemittel von einer leicht elastischen Struktur des Umhüllungsteils gebildet werden, wobei diese dann Abschnitte aufweist, die reibend auf dem Knochen aufliegen und/oder von mindestens zwei seitlichen Faserelementen (3) bildbar sind, die jeweils mit einem der Längsenden des Umhüllungsteils verbunden sind, wobei diese Faserelemente miteinander verbindbar sind, um den Sitz des Umhüllungsteil in Anwendungsstellung auf den Tuberositates zu gewährleisten.

12. Schulterprothese in Kombination mit der Osteosynthesevorrichtung nach Anspruch 11, **dadurch gekennzeichnet, dass** sie Mittel zur Befestigung mindestens eines der seitlichen Faserelemente (3), wie Rillen oder Öffnungen, umfasst.
